(19) Europäisches Patentamt
European Patent Office
Office européen des brevets

(11) **EP 2 497 536 A2**

(12) **EUROPÄISCHE PATENTANMELDUNG**

(43) Veröffentlichungstag:
**12.09.2012 Patentblatt 2012/37**

(21) Anmeldenummer: **12155054.5**

(22) Anmeldetag: **13.02.2012**

(51) Int Cl.:
**A61Q 5/08** (2006.01)     **A61K 8/22** (2006.01)
**A61K 8/41** (2006.01)     **A61K 8/23** (2006.01)
**A61K 8/46** (2006.01)     **A61K 8/43** (2006.01)
**A61K 8/24** (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(30) Priorität: **11.03.2011 DE 102011005430**

(71) Anmelder: **Henkel AG & Co. KGaA
40589 Düsseldorf (DE)**

(72) Erfinder:
• **Goutsis, Konstantin
41363 Jüchen (DE)**
• **Janßen, Frank
41470 Neuss (DE)**

(54) **Haaraufhellverfahren mit reduktiver Nachbehandlung**

(57)     Die vorliegende Anmeldung betrifft ein Verfahren zur Aufhell- und Blondierung keratinischer Fasern, welches durch eine zweistufige Anwendung von oxidativer Blondierzubereitung und reduktiver Nachbehandlung gekennzeichnet ist. Weiterhin wird eine Verpackungseinheit, umfassend solche Blondierzubereitungen und Nachbehandlungsmittel, offenbart.

EP 2 497 536 A2

**Beschreibung**

Die Erfindung betrifft mehrstufige Verfahren zum Blondieren und Aufhellen von keratinischen Fasern, insbesondere menschlicher Haare. Durch die sequentielle Anwendung oxidativer und reduktiver Anwendungsschritte wird dabei eine besonders effektive Aufhellleistung erzielt. Weiterhin betrifft die vorliegende Erfindung eine Verpackungseinheit, die eine oxidative Aufhell- und/oder Blondierzubereitung sowie ein reduktives Nachbehandlungsmittel enthält.

Die Veränderung von Form und Farbe der Haare stellt einen wichtigen Bereich der modernen Kosmetik dar und erlaubt die Anpassung des Erscheinungsbilds der Haare sowohl an aktuelle Modeströmungen als auch an individuelle Wünsche der einzelnen Person. Dabei können Dauerwell- und andere die Haarform verändernde Verfahren nahezu unabhängig vom Typ der zu behandelnden Haare eingesetzt werden. Dagegen sind Färbe- und Blondierverfahren auf bestimmte Ausgangshaarfarben begrenzt.

Das Aufhellen der eigenen Haarfarbe ist seit jeher der Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Im Nachfolgenden werden die Bezeichnungen Aufhellen und Blondieren nebeneinander als gleichbedeutend eingesetzt. Die in diesen aufhellenden oder blondierenden Produkten enthaltenen Oxidationsmittel sind in der Lage, die Haarfaser durch die oxidative Zerstörung des haareigenen Farbstoffes Melanin aufzuhellen. Für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Insbesondere bei dunklem Ausgangshaar, dessen Melanin-Zusammensetzung sowohl größere Anteile an braun-schwarzem Eumelanin als auch an gelblich-rötlichen Phämelanin besitzt, besteht das Problem, dass durch oxidative Aufhellmittel überwiegend Eumelanin zerstört und entfernt wird. Dies führt häufig zu unerwünschten Nuancierungen und wenig gefälligen Farbverschiebungen ins gelblich-rötliche. Um dem entgegen zu wirken, müssen Haare mit größeren Anteilen an Phämelanin üblicherweise wesentlich aufwendiger und durch längere Anwendungszeiten oder wiederholte Blondiervorgänge aufgehellt werden. Dadurch geht jedoch auch eine Schädigung des Haares einher, da nicht nur die Farbstoffe des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch.

Aus DE102008034845 A1 sind oxidative Aufhellmittel bekannt, bei denen Reduktionsmittel zugesetzt werden, um die Schädigung durch oxidative Einflüsse zu reduzieren. Aus DE 10152940 A1 sind Verfahren bekannt, bei denen künstliche Färbungen von Haaren mittels reduktiver Nachbehandlung rückgängig gemacht werden kann.

Aus dem Stand der Technik ist für den Fachmann nicht zu entnehmen, dass durch reduktive Nachbehandlung an ein oxidatives Aufhellverfahren die Aufhellleistung, insbesondere im Hinblick auf gelblich-rötliche Verschiebungen, verbessert werden könnte.

Aufgabe der vorliegenden Erfindung ist es daher, die genannten Nachteile von bekannten oxidativen Haaraufhell- und -blondierverfahren herabzusenken. Die Faserstruktur soll möglichst geschont werden, so dass ihre Elastizität möglichst wenig durch die Farbveränderungsmittel in Mitleidenschaft gezogen wird, und dass die Fasern eine möglichst geringe Verschlechterung der Faserstärke aufweisen. Insbesondere soll die Aufhellleistung insgesamt und insbesondere im Hinblick auf gelblich-rötliche Verschiebungen des Farbeindrucks, verbessert werden.

Es wurde nun in nicht vorhersehbarer Weise gefunden, dass die Faserstruktur von keratinhaltigen Fasern beim aufhellenden Verfahren in unerwartet weitem Umfang geschont wird und die Aufhellleistung, wenn zur Aufhellung und/oder Blondierung der keratinischen Fasern ein zweistufiges Verfahren eingesetzt wird, wobei zunächst ein Schritt zur oxidativen Aufhellung und/oder Blondierung erfolgt, und sich dann zeitnah ein Schritt anschließt, bei dem ein Nachbehandlungsmittel, enthaltend ein Reduktionsmittel, auf die Faser aufgebracht wird.

Dazu ist es bevorzugt, dem Anwender die einzelnen Komponenten zur Durchführung des erfindungsgemäßen Verfahrens getrennt konfektioniert in einer Mehrkomponentenverpackungseinheit bereit zu stellen.

Ein erster Gegenstand der vorliegenden Erfindung ist daher eine Mehrkomponentenverpackungseinheit (Kit-of-Parts), welche getrennt voneinander konfektioniert

(i) mindestens einen Container (I), enthaltend eine Oxidationsmittelzubereitung A, welche in einem kosmetisch verträglichen Träger mindestens Wasserstoffperoxid und/oder eines seiner Anlagerungsprodukte an organische oder anorganische Träger enthält,
(ii) mindestens einen Container (II), enthaltend mindestens eine Blondieraktivatorzubereitung B, welche mindestens ein Persalz, bevorzugt mindestens ein Persulfatsalz, enthält,
(iii) und mindestens einen Container (III), enthaltend mindestens ein Nachbehandlungsmittel C, welches in einem kosmetisch verträglichen Träger mindestens ein Reduktionsmittel enthält, umfasst.

Der Begriff "Container" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff "Container" umfasst daher - ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Komponenten der Zubereitungen können in einem einzelnen Container, der mehrere Kompartimente zur Aufnahme der Zubereitungen aufweist, enthalten sein, es ist aber auch möglich und gegebenenfalls bevorzugt, sie auf verschiedene Container aufzuteilen und den Verbraucher anzuleiten, sie vor der Anwendung miteinander zu mischen.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Der kosmetisch verträglichen Träger ist im Sinne der Erfindung wässrig, alkoholisch oder wässrigalkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.

Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten wesentlichen Inhaltsstoff enthält die Mehrkomponentenverpackungseinheit gemäß ersten Erfindungsgegenstand mindestens einen Container (I), der eine Oxidationsmittelzubereitung A enthält, welche in einem kosmetisch verträglichen Träger mindestens Wasserstoffperoxid und/oder eines seiner Anlagerungsprodukte an organische oder anorganische Träger enthält.

In einer bevorzugten Ausführungsform wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon n $H_2O_2$ (n ist eine positive ganze Zahl größer 0), Harnstoffperoxid und Melaminperoxid, eingesetzt werden. Im letztgenannten Fall setzen die Anlagerungsverbindungen in der erfindungsgemäßen Anwendungsmischung Wasserstoffperoxid frei, d. h. diese Mittel enthalten neben der Anlagerungsverbindung im kosmetischen Träger freies Wasserstoffperoxid.

Bevorzugt wird jedoch als Oxidationsmittel Wasserstoffperoxid als wässrige Lösung eingesetzt. Die Konzentration einer Wasserstoffperoxid-Lösung wird einerseits von den gesetzlichen Vorgaben und andererseits von dem gewünschten Effekt bestimmt; vorzugsweise werden 6 bis 12 gew.-%ige Lösungen in Wasser verwendet. Erfindungsgemäß bevorzugte Mittel sind dadurch gekennzeichnet, dass sie, bezogen auf ihr Gesamtgewicht, 0,01 bis 12 Gew.-%, vorzugsweise 0,1 bis 10 Gew.-%, besonders bevorzugt 1 bis 6 Gew.-% Wasserstoffperoxid (berechnet als 100%iges $H_2O_2$) enthalten.

Bevorzugte Oxidationsmittelzubereitungen A sind dabei dadurch gekennzeichnet, dass die Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Weiterhin hat es sich als vorteilhaft erwiesen, wenn die Oxidationsmittelzubereitung A mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Diese können unterschiedlichen chemischen Gruppen angehören. Vorzugsweise werden einzeln oder im Gemisch miteinander eingesetzt. Erfindungsgemäß bevorzugte Komplexbildner sind stickstoffhaltigen Polycarbonsäuren, insbesondere Ethylendiamintetraessigsäure (EDTA) und Ethylendiamindibernsteinsäure (EDDS), und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Als weiteren wesentlichen Inhaltsstoff enthält die Mehrkomponentenverpackungseinheit gemäß ersten Erfindungsgegenstand mindestens einen Container (II), der eine Blondieraktivatorzubereitung B enthält, welche mindestens ein Persalz, bevorzugt mindestens ein Persulfatsalz, enthält.

Vorzugsweise ist das Persalz der Blondieraktivatorzubereitung ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid.

Erfindungsgemäß besonders bevorzugt sind Blondieraktivatorzubereitungen B, die in der als Bleichkraftverstärker mindestens ein anorganisches Persalz, ausgewählt aus Peroxomonosulfaten und/oder Peroxodisulfaten (= Persulfaten), enthalten. Weiterhin hat es sich als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Eine weitere Ausführungsform dieses Erfindungsgegenstands ist daher dadurch gekennzeichnet, dass die Blondieraktivatorzubereitung B zusätzlich mindestens ein anorganisches Persulfat- oder Peroxodisulfat-Salz, insbesondere Ammoniumperoxodisulfat, Kaliumperoxodisulfat und/oder Natriumperoxodisulfat, enthält. Die Persalze sind in einer Menge von 5 bis 85 Gew.-%, insbesondere in einer Menge von 15 bis 75 Gew.-%, bezogen auf das Gesamtgewicht der Blondieraktivatorzubereitung B, enthalten.

Die erfindungsgemäßen Blondieraktivatorzubereitungen B können zusätzlich zu den Persalzen einen weiteren Bleichkraftverstärker enthalten.

Als weitere Bleichkraftverstärker können mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als weitere Bleichkraftverstärker vom Typ der Kohlensäurederivate können erfindungsgemäß weiterhin bevorzugt Carbonatsalze bzw. Hydrogencarbonatsalze eingesetzt werden. Diese sind bevorzugt ausgewählt aus der Gruppe der Ammonium-, Alkalimetall- (insbesondere Natrium- und Kalium-) sowie Erdalkalimetall- (insbesondere Magnesium- und Calcium-), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese bevorzugten Salze können allein oder in deren Mischungen von mindestens zwei Vertretern als Bleichverstärker verwendet werden.

Die neben Persalzen eingesetzten Bleichkraftverstärker sind in den Mitteln bevorzugt in Mengen von 0,5 bis 45 Gew.-%, insbesondere in Mengen von 2 bis 25 Gew.-%, jeweils bezogen auf das Gesamtgewicht der Blondieraktivatorzubereitung B, enthalten.

Zur weiteren Steigerung der Aufhellleistung kann der erfindungsgemäßen Blondieraktivatorzubereitung B daher zusätzlich mindestens eine gegebenenfalls hydratisierte $SiO_2$-Verbindung zugesetzt werden. Es kann erfindungsgemäß bevorzugt sein, die gegebenenfalls hydratisierten $SiO_2$-Verbindungen in einem Anteil von 0,5 Gew.-% bis 50 Gew.-%, besonders bevorzugt in Mengen von 1,0 Gew.-% bis 25 Gew.-% und ganz besonders bevorzugt in Mengen von 2 Gew.-% bis 20 Gew.-%, jeweils bezogen auf die erfindungsgemäße Blondieraktivatorzubereitung B, einzusetzen. Die Mengenangaben geben dabei jeweils den Gehalt der $SiO_2$-Verbindungen (ohne deren Wasseranteil) in den Mitteln wieder.

Hinsichtlich der gegebenenfalls hydratisierten $SiO_2$-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten $SiO_2$-Verbindungen können in verschiedenen Formen vorliegen. Erfindungsgemäß bevorzugt werden die $SiO_2$-Verbindungen in Form von Kieselgelen (Silicagel) oder besonders bevorzugt als Wasserglas eingesetzt. Diese $SiO_2$-Verbindungen können teilweise in wässriger Lösung vorliegen.

Erfindungsgemäß ganz besonders bevorzugt sind Wassergläser, die aus einem Silicat der Formel $(SiO_2)_n(Na_2O)_m(K_2O)_p$ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 2:1 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem unter den Bezeichnungen Ferrosil® 119, Natronwasserglas 40/42, Portil® A, Portil® AW und Portil® W und unter der Bezeichnung Britesil® C20 vertrieben.

Erfindungsgemäß weiterhin ganz besonders bevorzugt sind Metasilicate, die aus einem Silicat der Formel $(SiO_2)_n(Na_2O)_m(K_2O)_p$ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p von kleiner oder gleich 1 liegt.

Die Blondieraktivatorzubereitung B wird in der Regel in Form einer wasserfreien Zubereitung, insbesondere eines gegebenenfalls entstaubten Pulvers, einer Paste oder eines in Form gepressten Formkörpers eingesetzt.

Wasserfrei im Sinne der vorliegenden Erfindung bedeutet einen Wassergehalt bezogen auf die Blondieraktivatorzubereitung B von weniger als 5 Gew.-%, insbesondere von weniger als 2 Gew.-%. Blondieraktivatorzubereitungen B, die weniger als 0,1 Gew.-% Wasser, enthalten können erfindungsgemäß ganz besonders bevorzugt sein. Die Blondieraktivatorzubereitung B ist vorzugsweise wasserfrei als Pulver oder als Paste formuliert.

Im Falle einer wasserfreien Formulierung hat es sich als besonders bevorzugt erwiesen, wenn die Blondieraktivatorzubereitung B mindestens einen nicht hydroxylierten Fettsäureester mit einem Schmelzpunkt von höchstens 50°C, insbesondere von höchstens 30°C, und/oder mindestens eine Cis-$C_{30}$-Fettsäure mit mindestens einer zusätzlichen Hydroxygruppe und/oder ein Derivat davon, enthält. Ester von nicht hydroxylierten $C_6$-$C_{30}$-Alkylmonocarbonäuren mit $C_2$-$C_{30}$-Monoalkoholen eignen sich erfindungsgemäß bevorzugt als Fettsäureester. Erfindungsgemäß besonders bevorzugt sind Isopropylmyristat, Isononansäure-cetearylester, 2-Ethylhexylpalmitat, Stearinsäure-2-ethylhexylester, Cetyloleat, Kokosfettalkohol-caprinat/-caprylat, n-Butylstearat, Oleylerucat, Isopropylpalmitat, Oleyloleat, Laurinsäurehexylester, Myristylmyristat, Cetearylisononanoat, Ölsäuredecylester.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des verwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Als Metallionen dienen bevorzugt Magnesium, Natrium und/oder Kalium. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Bevorzugte Alkanolamine sind Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Es hat sich im Laufe der Untersuchungen der Anmelderin herausgestellt, dass sich die Blondierleistung der Mittel weiterhin steigern lässt, wenn die Aufhellmittel mindestens ein aromatisches, organisches Lösungsmittel enthalten. Aromatische Lösungsmittel im Sinne der Erfindung sind dabei solche Verbindungen, die eine aromatische Struktureinheit, wie beispielsweise eine Phenylgruppe, in ihrer Strukturformel aufweisen und weiterhin unter Normalbedingungen, also Raumtemperatur und Normaldruck, flüssig sind. Vorzugsweise handelt es sich dabei um carbocyclische Lösungsmittel, die bevorzugt zusätzlich eine Hydroxygruppe tragen. Bevorzugte Beispiele für solche aromatischen Lösungsmittel sind Alkohole, wie Benzylalkohol, 2-Phenylethylalkohol, 1-Phenylethylalkohol, 2-Phenoxyethanol, 3-Methylbenzylalkohol, 2-Methoxybenzylalkohol und 3-Methoxybenzylalkohol.

Ein erfindungsgemäß ganz besonders bevorzugtes aromatisches Lösungsmittel ist Benzylalkohol. Es sind dabei erfindungsgemäße Mittel bevorzugt, die 0,01 bis 15 Gew.-%, insbesondere 0,1 bis 10 Gew.-% und besonders bevorzugt 0,5 bis 5,0 Gew.-%, jeweils bezogen auf das Gesamtgewicht des anwendungsbereiten Mittels, mindestens eines aromatischen, organischen Lösungsmittels enthalten.

Die erfindungsgemäßen Oxidationsmittelzubereitung A und Blondieraktivatorzubereitung B werden vor der Anwendung miteinander vermischt und das resultierende Blondiermittel auf die keratinischen Fasern aufgetragen.

Die resultierenden Blondiermitteln weisen bevorzugt einen pH-Wert im Bereich von 7 bis 12 aufweist. Im Fall von Oxidationsfärbemitteln findet die Anwendung der Färbemittel in einem schwach alkalischen Milieu statt, bevorzugt bei einem pH-Wert im Bereich von 8,0 bis 11,5.

Bei den pH-Werten im Sinne der vorliegenden Erfindung handelt es sich um pH-Werte, die bei einer Temperatur von 22 °C gemessen wurden.

Je nach Anwendung kann es daher bevorzugt sein, wenn das resultierende Blondiermittel noch aus einer weiteren Komponente zusammengemischt wird, die ein Alkalisierungsmittel enthält.

Eine weitere Ausführungsform des ersten Erfindungsgegenstands ist daher eine Verpackungseinheit (Kit-of-Parts), die dadurch gekennzeichnet ist, dass die Verpackungseinheit zusätzlich

(iv) mindestens einen Container (IV) umfasst, enthaltend eine Alkalisierungszubereitung D, welche in einem kosmetisch verträglichen Träger mindestens ein Alkalisierungsmittel, ausgewählt aus Ammoniak und/oder Alkanolaminen, enthält.

Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethylethanolamin, Methylglucamin, Triethanolamin, Diethanolamin und Triisopropanolamin. Erfindungsgemäß ganz besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Insbesondere bevorzugte Alkanolamine sind Monoethanolamin, und Triethanolamin. Schließlich ist ein weiteres bevorzugtes Alkalisierungsmittel Ammoniak.

Das anwendungsbereite Blondiermittel kann weitere Alkalisierungsmittel enthalten.

Erfindungsgemäß einsetzbare, weitere Alkalisierungsmittel sind anorganische Alkalisierungsmittel, bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Ganz besonders bevorzugt sind Natriumhydroxid und/oder Kaliumhydroxid. Als weitere Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt.

Als weitere wesentliche Komponente enthält die erfindungsgemäße Verpackungseinheit (Kit-of-Parts) mindestens einen Container (III), enthaltend mindestens ein Nachbehandlungsmittel C, welches in einem kosmetisch verträglichen Träger mindestens ein Reduktionsmittel enthält,

Das Nachbehandlungsmittel C enthält als wesentlichen Inhaltstoff mindestens ein Reduktionsmittel. Hierzu zählen insbesondere physiologisch verträgliche chemische Reduktionsmittel.

In einer bevorzugten Ausführungsform des ersten Erfindungsgegenstands ist das Reduktionsmittel im Nachbehandlungsmittel C ausgewählt aus anorganischen und organischen Schwefel-haltigen Reduktionsmitteln.

Zu organischen Schwefel-haltigen Reduktionsmitteln zählen insbesondere Schwefel-haltige, organische Säuren, wie Thiomilchsäure, Thioglycolsäure und/oder einem ihrer physiologisch verträglich Salze, und organische Disulfide.

Bevorzugte Schwefel-haltige, organische Säure ist Thiomilchsäure, besonders bevorzugt ist Natriumthiolactat und insbesondere Ammoniumthiolactat (ATL).

Erfindungsgemäß einsetzbare, organische Disulfide können linear oder cyclisiert sein. Beispiele für lineare Disulfide sind Cystin, Di-*t*-butyl-disulfid, Dibenzyldisulfid oder Difurfuryldisulfid. Erfindungsgemäß besonders vorteilhaft ist die Verwendung von cyclischen Disulfiden als funktionaler Additivstoff. Als bevorzugtes Beispiel ist insbesondere α-Liponsäure zu nennen.

Die erfindungsgemäß geeigneten anorganischen Reduktionsmittel sind insbesondere wasserlösliche Salze von Alkali- und/oder Erdalkalimetallen und Schwefel-haltigen Anionen. Bevorzugte Kationen sind hierbei Natrium, Kalium und Magnesium. Als bevorzugte Anionen sind Sulfid ($S^{2-}$), Hydrogensulfid ($HS^-$), Sulfit ($SO_3^{2-}$), Hydrogensulfit ($HSO_3^-$), Thiosulfat ($S_2O_3^{2-}$), Dithionit ($S_2O_4^{2-}$) und Disulfit ($S_2O_5^{2-}$) zu erwähnen. Besonders bevorzugt ist hierbei Natriumdithionit.

In einer weiteren bevorzugten Ausführungsform des ersten Erfindungsgegenstands ist das Reduktionsmittel im Nachbehandlungsmittel C ausgewählt aus Salzen der Thiomilchsäure, insbesondere Ammoniumthiolactat, und Salzen der Dithionigen Säure, insbesondere Natriumdithionit.

Eine weitere bevorzugten Ausführungsform des ersten Erfindungsgegenstands ist dadurch gekennzeichnet, dass das Reduktionsmittel im Nachbehandlungsmittel C in einem Anteil von 0,1 bis 15 Gew.-%, bevorzugt 0,5 bis 12 Gew.-%, besonders bevorzugt 1,0 bis 10 Gew.-% und insbesondere 2,0 bis 8,0 Gew.-%, jeweils bezogen auf das Gesamtgewicht des anwendungsbereiten Nachbehandlungsmittels C, enthalten ist.

Das Nachbehandlungsmittel C kann weitere Inhalts- und Wirkstoffe besitzen. Insbesondere wurde gefunden, dass bestimmte weitere Zusätze einen zusätzlichen Effekt auf die Blondierleistung haben können.

Eine weitere bevorzugte Ausführungsform des ersten Erfindungsgegenstands ist dadurch gekennzeichnet, dass in der Mehrkomponentenverpackungseinheit das Nachbehandlungsmittel C zusätzlich mindestens Harnstoff enthält.

Besonders bevorzugt enthält das Nachbehandlungsmittel C Harnstoff in einem Anteil von 0,1 bis 15 Gew.-%, bevorzugt 0,5 bis 12 Gew.-%, besonders bevorzugt 1,0 bis 10 Gew.-% und insbesondere 2,0 bis 8,0 Gew.-%, jeweils bezogen auf das Gesamtgewicht des anwendungsbereiten Nachbehandlungsmittels C.

Das Nachbehandlungsmittel kann weiterhin konditionierende Wirk- und Pflegestoffe enthalten. Dazu zählen erfindungsgemäß Öle und Silicone, nichtsubstituierte oder substituierte, nicht ionische oder ionische Proteinhydrolysate, kationische Polymere und kationische Tenside.

Erfindungsgemäß bevorzugt sind kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Eine erfindungsgemäß besonders geeignetes Alkylamidoamin stellt das unter der Bezeichnung Tegoamid® S 18 im Handel erhältliche Stearamidopropyldimethylamin dar.Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte N,N-Bis-(2-Palmitoyloxyethyl)dimethylammoniumchlorid, sowie Dehyquart® F-75, Dehyquart®C-4046, Dehyquart® L80 und Dehyquart® AU-35 sind ebenfalls Beispiele für solche Esterquats.

Die kationischen Tenside sind in den erfindungsgemäß verwendeten Nachbehandlungsmittel C bevorzugt in Mengen von 0,05 bis 10 Gew.-%, bezogen auf das gesamte Mittel, enthalten. Mengen von 0,1 bis 5 Gew.-% sind besonders bevorzugt.

Als weitere konditionierende Wirk- und Pflegestoffe können bevorzugt kationische Polymere, wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol, haarkonditionierende Verbindungen wie Phospholipide, beispielsweise Sojalecithin, Ei-Lecitin und Kephaline sowie Silikonöle, und Proteinhydrolysate pflanzlicher oder tierischer Herkunft, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate in das Nachbehandlungsmittel C eingearbeitet werden.

Der erfindungsgemäß bevorzugte pH-Wert der Nachbehandlungsmittel C liegt in einem sauren pH-Bereich.

Eine weitere bevorzugte Ausführungsform des ersten Erfindungsgegenstands ist dadurch gekennzeichnet, dass in der Mehrkomponentenverpackungseinheit das Nachbehandlungsmittel C einen pH-Wert von 1,5 bis 7,0, bevorzugt 2,5 bis 6,0 und besonders bevorzugt von 3,0 bis 5,5 aufweist.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Ferner können die Zubereitungen der erfindungsgemäßen Mehrkomponentenverpackungseinheit weitere Wirk-, Hilfs- und Zusatzstoffe enthalten.

Bevorzugt enthalten die Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, zwitterionischen und nichtionischen Tensiden und Emulgatoren ausgewählt sind.

Erfindungsgemäß bevorzugte Zubereitungen sind dadurch gekennzeichnet, dass die Zubereitung zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Mengen von 0,1 bis 45 Gew.%, bevorzugt 1 bis 30 Gew.% und ganz besonders bevorzugt von 1 bis 15 Gew.%, jeweils bezogen auf das Gesamtgewicht der anwendungsbereiten Zubereitung, eingesetzt.

Erfindungsgemäß bevorzugte Zubereitungen sind dadurch gekennzeichnet, dass die Zubereitung zusätzlich mindestens ein zwitterionisches Tensid enthält. Besonders geeignete zwitterionische Tenside sind die sogenannten Betaine und N-Alkyl-N,N-dimethylammonium-glycinate, N-Acyl-amino-propyl-N,N-dimethylammoniumglycinate, und 2-Alkyl-3-carboxymethyl-3-hydroxyethyl-imidazoline. Ein bevorzugtes zwitterionisches Tensid ist das unter der INCI-Bezeichnung Cocamidopropyl Betaine bekannte Fettsäureamid-Derivat. Erfindungsgemäß bevorzugte Zubereitungen sind dadurch gekennzeichnet, dass die Zubereitung zusätzlich mindestens ein amphoteres Tensid enthält. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind das N-Kokosalkylaminopropionat, das Kokosacylaminoethylaminopropionat und das $C_{12}$-$C_{18}$-Acylsarcosin. Weiterhin hat es sich als vorteilhaft erwiesen, wenn die Zubereitungen weitere, nichtionogene grenzflächenaktive Stoffe, enthalten. Als bevorzugte nichtionische Tenside haben sich Alkylpolyglykoside sowie Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in einem Anteil von 0,1 bis 45 Gew.%, bevorzugt 1 bis 30 Gew.% und ganz besonders bevorzugt von 1 bis 15 Gew.%, bezogen auf das Gesamtgewicht der Zubereitungen, eingesetzt.

Die anwendungsbereiten Zubereitungen können weitere Hilfs- und Zusatzstoffe enthalten. So hat es sich als vorteilhaft erwiesen, wenn die Zubereitungen mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind insbesondere

- anionische, synthetische Polymere anionische Polymere wie beispielsweise Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/ Isobornylacrylat-Copolymere, Methylvinylether/Maleinsäureanhydrid-Copolymere und Acrylsäure/Ethylacrylat/N-tert-Butyl-acrylamid-Terpolymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol, Polyvinylpyrrolidinon, Vinylpyrrolidinon/Vinylacrylat-Copolymere, Vinylpyrrolidinon/Vinylacetat-Copolymere, Polyethylenglykole und Polysiloxane; sowie
- anorganische Verdickungsmittel, insbesondere Schichtsilikate wie beispielsweise Bentonit, besonders Smektite, wie Montmorillonit oder Hectorit.

Weiterhin können die Aufhellmittel zur Mattierung von unerwünschten Restfarbeindrücken, insbesondere im rötlichen oder bläulichen Bereich, bestimmte, direktziehende Farbstoffe der komplementären Farben enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe werden jeweils bevorzugt in einer Menge von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Ganz besonders bevorzugt sind Aufhellmittel, die mindestens eine Kombination aus Tetrabromphenolblau und Acid Red 92 enthalten.

Ferner können die erfindungsgemäßen Zubereitungen weitere Wirk-, Hilfs- und Zusatzstoffe enthalten, wie beispielsweise

- Strukturanten wie Glucose, Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,

- faserstrukturverbessernde Wirkstoffe, insbesondere Mono-, Di- und Oligosaccharide wie beispielsweise Glucose, Galactose, Fructose, Fruchtzucker und Lactose,
- Entschäumerwie Silikone, bevorzugt Dimethicon,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Wirkstoffe wie Allantoin, Pyrrolidinoncarbonsäuren und deren Salze sowie Bisabolol,
- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- pflanzliche Öle, wie beispielsweise Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, $N_2O$, Dimethylether, $CO_2$ und Luft,
- Antioxidantien.

Die Auswahl dieser weiteren Stoffe wird der Fachmann gemäß den gewünschten Eigenschaften der Zubereitungen treffen. Die erfindungsgemäß eingesetzten Zubereitungen enthalten die weitere Wirk-, Hilfs- und Zusatzstoffe bevorzugt in Mengen von 0,1 bis 25 Gew.-%, insbesondere 0,5 bis 15 Gew.-%, bezogen auf die Gesamtmenge des anwendungsbereiten Mittel.

Die Zubereitungen der erfindungsgemäßen Verpackungseinheit des ersten Erfindungsgegenstands werden bevorzugt in einem mehrstufigen Verfahren eingesetzt.

Ein weiterer Gegenstand der vorliegenden Erfindung ist daher ein Verfahren zur Aufhellung keratinischer Fasern, bei dem in einem ersten Schritt eine Aufhell- und/oder Blondierzubereitung auf die Fasern aufgetragen wird, für eine Einwirkzeit T1 auf den Fasern belassen wird und anschließend mit Wasser ausgewaschen wird, und in einem zweiten Schritt ein Nachbehandlungsmittel D, welches in einem kosmetisch verträglichen Träger mindestens ein Reduktionsmittel enthält, auf die keratinischen Fasern aufgetragen wird, für eine Einwirkzeit T2 auf den Fasern belassen wird und anschließend ausgewaschen wird,
dadurch gekennzeichnet, dass

a) die Aufhell- und/oder Blondierzubereitung unmittelbar vor der Anwendung durch Vermischen der Oxidationsmittelzubereitung A und der Blondieraktivatorzubereitung B sowie gegebenenfalls der Alkalisierungszubereitung D gemäß dem ersten Erfindungsgegenstand hergestellt wird und

b) der Zeitraum zwischen Beendigung des ersten Verfahrensschritt und Beginn des zweiten Verfahrensschritts maximal 60 Minuten beträgt.

Die bevorzugten Ausführungsformen des ersten Erfindungsgegenstands gelten mutatis mutandis für das erfindungsgemäße Verfahren des zweiten Erfindungsgegenstandes.

Im ersten Schritt des erfindungsgemäßen Verfahrens wird eine Blondierzubereitung auf die keratinischen Fasern aufgetragen und für eine bestimmte Einwirkzeit im Haar belassen. Die Auftragung der Blondierzubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber ebenso möglich, die Blondierzubereitung mit einer Applikationshilfe auf die keratinischen Fasern aufzutragen. Als Applikationshilfen eignen sich insbesondere Kamm, Bürste, Pinsel, Mascara-Bürste oder Applicette.

Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

In einem bevorzugten Anwendungsverfahren werden die Aufhell- und/oder Blondierzubereitungen bevorzugt mittels einer Folie aufgebracht.

Eine bevorzugte Ausführungsform des zweiten Erfindungsgegenstands ist ein Verfahren, welches dadurch gekennzeichnet ist, dass für den ersten Schritt des Verfahrens die anwendungsbereite Aufhell- und/oder Blondierzubereitung auf einer oder mehreren Folien, geeignet zur Einschlagung einer oder mehrer Bündel keratinischer Fasern, aufgebracht ist, diese Folie und/oder Folien um ein oder mehrere Bündel keratinischer Fasern geschlagen werden, für die Einwirkzeit T1 um den Bündeln belassen werden und anschließend vor dem Auswaschen der keratinischen Fasern entfernt werden.

Dabei wird eine Folie, auch so genannte Strähnchenfolien oder Strähnchenpapiere zur Erstellung von Strähnchen einzusetzen. Dabei handelt es sich um flexible Substrate, die mit der anwendungsbereiten Blondierzubereitung beaufschlagt werden. Die beaufschlagten Substrate werden zur Anwendung um die aufzuhellende Strähne gewickelt. Dadurch lassen sich selektiv und je nach Wunsch des Anwenders bestimmte Partien von menschlichem Haupthaar auswählen und aufhellen.

Die Auftragungs- und die Einwirktemperatur der Blondierzubereitung beträgt Raumtemperatur bis 45°C. Gegebenenfalls kann die Wirkung der Färbezubereitung durch externe Wärmezufuhr, wie beispielsweise mittels einer Wärmehaube, verstärkt werden.

Die bevorzugte Einwirkungsdauer T1 der Blondierzubereitung auf die keratinische Faser beträgt 2 bis 60 Minuten, bevorzugt 15 bis 45 Minuten.

Nach Beendigung der Einwirkdauer T1 wird die verbliebene Blondierzubereitung aus den keratinischen Fasern mit Hilfe von Wasser ausgewaschen.

Hierbei eignet sich vorzugsweise warmes Wasser, welches zusätzlich gegebenenfalls oberflächenaktive Zusätze (Tenside) enthält. Solche Tenside werden untenstehend beschrieben. Die bevorzugte Anwendungstemperatur von Wasser liegt insbesondere bei 25°C bis 60°C, vorzugsweise bei 30°C bis 55°C.

Nach dem Auswaschen werden die keratinischen Fasern gegebenenfalls mit einem Handtuch oder einem Heißluftgebläse (Fön) getrocknet. Bevorzugt wird, insbesondere bei langem Strähnen, mit handtuchfeuchten Strähnen gearbeitet.

Es hat sich gezeigt, dass die angestrebte verbesserte Aufhellleistung bevorzugt dann erreichbar ist, wenn sich der zweite Verfahrensschritt möglichst zeitnah nach Beendigung des ersten Verfahrensschritts anschließt. Wird der zweite Verfahrensschritt erst mit einer Verzögerung von einigen Stunden oder gar Tagen durchgeführt, so ist kein signifikant positiver Effekt bezüglich der Aufhellleistung mehr erkennbar.

Der Zeitraum, der nach Beendigung des ersten Verfahrensschritts bis zu Beginn des zweiten Verfahrensschritts liegt, beträgt daher maximal 60 Minuten, bevorzugt maximal 30 Minuten und insbesondere bevorzugt maximal 10 Minuten. Es kann bevorzugt sein, den zweiten Verfahrensschritt unmittelbar nach Beendigung des ersten Verfahrensschritts zu beginnen.

Im zweiten Schritt des erfindungsgemäßen Verfahrens wird ein Nachbehandlungsmittel D gemäß dem ersten Erfindungsgegenstand auf die keratinischen Fasern aufgetragen und für eine bestimmte Einwirkzeit T2 im Haar belassen.

Die Auftragungs- und die Einwirktemperatur des Nachbehandlungsmittels beträgt Raumtemperatur bis 45°C. Gegebenenfalls kann die Wirkung des Nachbehandlungsmittels durch externe Wärmezufuhr, wie beispielsweise mittels einer Wärmehaube, verstärkt werden.

Die bevorzugte Einwirkungsdauer T2 des Nachbehandlungsmittels auf die keratinischen Fasern beträgt 2 bis 25 Minuten, bevorzugt 5 bis 15 Minuten.

Nach Beendigung der Einwirkdauer T2 wird das verbliebene Nachbehandlungsmittel aus den keratinischen Fasern mit Hilfe einer Reinigungszubereitung ausgewaschen.

Als Reinigungszubereitung eignet sich hierbei vorzugsweise warmes Wasser, welches zusätzlich gegebenenfalls oberflächenaktive Zusätze (Tenside) enthält. Solche Tenside werden untenstehend beschrieben. Die bevorzugte Anwendungstemperatur der Reinigungszubereitung liegt insbesondere bei 25°C bis 60°C, vorzugsweise bei 30°C bis 55°C.

Nach dem Auswaschen werden die keratinischen Fasern gegebenenfalls mit einem Handtuch oder einem Heißluftgebläse getrocknet.

Es hat sich gezeigt, dass sich die Aufhellleistung oxidativer Blondier- und Aufhellmittel auf keratinischen Fasern signifikant erhöhen lässt, wenn ein Reduktionsmittel-haltiges Nachbehandlungsmittel eingesetzt wird.

Ein weiterer Gegenstand der Erfindung ist schließlich die Verwendung von Reduktionsmitteln in Nachbehandlungsmitteln zur Verstärkung der Aufhellleistung bei oxidativen Aufhell- und Blondierverfahren keratinischer Fasern.

Bezüglich weiterer bevorzugter Ausführungsformen der erfindungsgemäßen Verwendungen gilt mutatis mutandis das zu den erfindungsgemäßen Verpackungseinheiten und Verfahren Gesagte.

Beispiele

1. Zubereitungen

1.1 Herstellung der Oxidationszubereitung A

Aus den aufgelisteten Bestandteilen (Angaben in Gew.-%) wurden Oxidationszubereitungen wie folgt hergestellt:

|  | A1 |
|---|---|
| Ammoniak, 25 % | 0,62 |
| Dipicolinsäure | 0,10 |
| Dinatriumpyrophosphat | 0,03 |
| HEDP, 60% | 1,5 |
| Natrium Laurethsulfat (2 EO) | 0,54 |
| Dow Corning DB 110 A | 0,07 |
| Aculyn 33A | 15,00 |
| Wasserstoffperoxid, 50 % | 22,40 |
| Wasser, vollentsalzt | ad 100 |

Aculyn 33 A            ca. 28% Festkörper in Wasser; INCI-Bezeichnung-. Acrylates Copolymer (Rohm & Haas)

Dow Corning® DB 110 A     INCI-Bezeichnung: Dimethicon (Dow Corning)

1.2 Herstellung der Blondieraktivatorzubereitung B

Aus den aufgelisteten Bestandteilen (Angaben in Gew.-%) wurden Blondieraktivatorzubereitungen wie folgt hergestellt:

|  | B1 |
|---|---|
| Ammoniumpersulfat | 12,48 |
| Silica | 0,52 |
| Britesil C 20 | 28,00 |
| Kaliumpersulfat | 42,80 |
| Natriumpersulfat | 15,60 |
| EDTA Na2 | 0,600 |

Britesil C 20 INCI-Bezeichnung: Sodium Silicate 1.3 Herstellung der Alkalisierungszubereitung D

Aus den aufgelisteten Bestandteilen (Angaben in Gew.-%) wurden Alkalisierungszubereitungen wie folgt hergestellt:

|  | D1 |
|---|---|
| Cetearyl alcohol | 11,00 |
| Coconut alcohol | 2,75 |
| Ceteareth-20 | 0,25 |
| Ammoniumsulfat | 1,00 |
| Natrium Laurethsulfat (2 EO) | 7,55 |
| HEDP, 60% | 0,20 |
| Natriumsilikat 40/42 | 0,50 |
| Gluadin W40 | 0,35 |
| Vitamin F Forte | 1,00 |

(fortgesetzt)

|  | D1 |
|---|---|
| Ammoniak 25 % | 7,60 |
| Wasser, vollentsalzt | ad 100 |

Gluadin W 40    ca. 40 % Aktivsubstanz, INCI-Bezeichnung: Hydrolyzed Wheat Protein (Cognis)

Vitamin F Forte    Fettsäuregemisch, ca. 75 % Linolsäure, INCI-Bezeichnung: Linoleic Acid, Linolenic Acid (CLR Kurt Richter)

Die Mittel A1, B1 und D1 wurden im Verhältnis 1: 0,26: 0,95 miteinander vermischt und so die anwendungsbereite Blondierzubereitung erhalten.

1.4 Nachbehandlungsmittel C

Aus den aufgelisteten Bestandteilen (Angaben in Gew.-%) wurden Nachbehandlungsmittel C wie folgt hergestellt:

| Rohstoff | C1 | C2 | C3 | C4 | C5 |
|---|---|---|---|---|---|
| Isopropylmyristat | 1,00 | 1,30 | 1,30 | 1,30 | 1,30 |
| Cutina GMS-V | 0,80 | 0,30 | 0,30 | 0,30 | 0,30 |
| Ceterayl Alcohol | 5,1 | 4,60 | 4,60 | 4,60 | 4,60 |
| Dehyquart F75 | 1,0 | 1,00 | 1,00 | 1,00 | 1,00 |
| Steraramidopropyldimethylamine | 0,8 | 0,40 | 0,40 | 0,40 | 0,40 |
| Methylparaben | 0,2 | 0,30 | 0,30 | 0,30 | 0,30 |
| Citronensäure, Monohydrat | 0,55 | 0,45 | 0,45 | 0,45 | 0,45 |
| Natriumdithionit | - | - | - | 5,00 | 5,00 |
| Ammoniumthiolactat | - | 3,50 | 3,50 | - | - |
| Harnstoff | - | - | 2,30 | - | 2,30 |
| Phenoxyethanol | 0,40 | 0,40 | 0,40 | 0,40 | 0,40 |
| Wasser | ad 100 | ad 100 | ad 100 | ad 100 | ad 100 |

Cutina® GMS-V    Glycerin Mono-/Di-Palmitat/Stearat (INCI-Bezeichnung: Glyceryl Stearate) (Cognis)

Dehyquart® F 75    Di(stearoylethyl)-(2-hydroxyethyl)-methyl-ammonium methylsulfat (ca. 69% Aktivsubstanz in Cetearylalkohol; INCI-Bezeichnung: Distearoylethyl hydroxyethylmonium methosulfate, Cetearyl alcohol) (Cognis)

2. Applikation Für den Blondierung wurde auf Strähnen (Code: Kerling 6/0 dunkles europäisches Haar) von ca. 1 g und von ca. 7 cm Länge jeweils die 4-fache Menge der fertigen Blondierzubereitung appliziert. Nachdem die Strähnen für die jeweils angegebene Zeit bei 32°C belassen wurden, wurden sie mit einem warmen Wasser (32°C) ausgewaschen.

Unmittelbar anschließend wurden 0,5 g der Nachbehandlungsmittel C1 bis C5 auf die Strähnen aufgetragen, für 10 min einwirken gelassen und anschließend mit warmen Wasser (32°C) ausgewaschen und mit einem Fön getrocknet.

Der Blondiervorgang und Nachbehandlungsvorgang wurde ggf. entsprechend der Angabe aus Tabelle 5 wiederholt.

Jede Haarsträhne wurde vor und nach dem Bleichvorgang farbmetrisch mit einem Farbmessgerät der Firma Datacolor, Typ Spectraflash 450 vermessen. Als Maß für die Aufhellleistung der jeweiligen Rezeptur wurde der ΔL-Wert gemäß folgender Formel herangezogen:

$\Delta L = L_{nachher} - L_{vorher}$

$L_{nachher}$ = Helligkeit der Strähne nach dem Bleichen

$L_{vorher}$ = Helligkeit der Strähne vor dem Bleichen

Für jede Rezeptur und jeden Haartyp erfolgte eine Doppelbestimmung, aus den Einzelwerten wurde jeweils der Mittelwert gebildet. Je größer der ∆L-Wert, desto besser ist die Aufhellleistung der jeweiligen Rezeptur.

Die Ergebnisse der Messungen wurden mithilfe des CIELAB Farbenraums quantifiziert.

Der L-Wert steht dabei für die Helligkeit der Färbung (schwarz-weiß-Achse); je größer der Wert für L ist, desto heller ist die Färbung. Der a-Wert steht für die rot-grün-Achse des Systems; je größer der Wert ist, umso mehr ist die Färbung ins Rote verschoben. Der b-Wert steht für die gelb-blau-Achse des Systems; je größer der Wert ist, umso mehr ist die Färbung ins Gelbe verschoben. Farbunterschiede werden mittels des ∆E-Wertes charakterisiert, der entsprechend Gleichung (I) berechnet wird:

$$\Delta E = \sqrt{(\Delta L)^2 + (\Delta a)^2 + (\Delta b)^2} \quad \text{(I)}$$

Als nicht erfindungsgemäßer Vergleich (C1) wurden entsprechende Haarsträhnen mit der gleichen Nachbehandlungszubereitung ohne erfindungsgemäße Reduktionsmittel behandelt.

Tabelle 4. Ergebnisse

| Aufhellung | Nachbehandlung | Nachbehandlungszubereitung | L | a | b | ∆E | ∆L |
|---|---|---|---|---|---|---|---|
| -- | -- | -- | 25,1 | 5,7 | 8,6 | -- | -- |
| 45 min | 10 min | C1 | 55,0 | 11,1 | 32,4 | 38,6 | 29,9 |
| 1x 45 min + 2x 20 min | 3x10 min | C1 | 61,7 | 9,3 | 32,0 | 43,6 | 36,6 |
| 45 min | 10 min | C2 | 57,0 | 10,6 | 32,5 | 40,2 | 32,0 |
| 1x45 min + 2x20 min | 3x10 min | C2 | 63,2 | 8,8 | 31,2 | 44,5 | 38,2 |
| 45 min | 10 min | C3 | 55,6 | 10,8 | 31,7 | 38,7 | 30,6 |
| 1x45 min + 2x20 min | 3x10 min | C3 | 63,0 | 9,0 | 31,0 | 44,2 | 38,0 |
| 45 min | 10 min | C4 | 54,8 | 11,5 | 33,1 | 38,9 | 29,7 |
| 1x45 min + 2x20 min | 3x10 min | C4 | 65,0 | 8,2 | 30,6 | 45,7 | 40,0 |
| 45 min | 10 min | C5 | 56,8 | 10,5 | 32,6 | 40,1 | 31,8 |
| 1x45 min + 2x20 min | 3x10 min | C5 | 66,1 | 7,7 | 29,7 | 46,1 | 41,0 |

Aus der stärkeren Aufhellleistung (∆L) und dem größeren Farbabstand (∆E) für die erfindungsgemäßen Verfahren mit erfindungsgemäßen Nachbehandlungsmitteln (C2 bis C5) sind die technischen Vorteile gegenüber dem nicht erfindungsgemäßen Verfahren (mit C1) deutlich.

**Patentansprüche**

**1.** Mehrkomponentenverpackungseinheit (Kit-of-Parts), welche getrennt voneinander konfektioniert

i. mindestens einen Container (I), enthaltend eine Oxidationsmittelzubereitung A, welche in einem kosmetisch verträglichen Träger mindestens Wasserstoffperoxid und/oder eines seiner Anlagerungsprodukte an organische oder anorganische Träger enthält,
ii. mindestens einen Container (II), enthaltend mindestens eine Blondieraktivatorzubereitung B, welche mindestens ein Persalz enthält,
iii. und mindestens einen Container (III), enthaltend mindestens ein Nachbehandlungsmittel C, welches in einem kosmetisch verträglichen Träger mindestens ein Reduktionsmittel enthält,
umfasst.

**2.** Verpackungseinheit (Kit-of-Parts) nach Anspruch 1, **dadurch gekennzeichnet, dass** die Verpackungseinheit zusätzlich

(iv) mindestens einen Container (IV) umfasst, enthaltend eine Alkalisierungszubereitung D, welche in einem kosmetisch verträglichen Träger mindestens ein Alkalisierungsmittel, ausgewählt aus Ammoniak und/oder Alkanolaminen, enthält.

3. Verpackungseinheit (Kit-of-Parts) nach einem der Ansprüche 1 bis 2, **dadurch gekennzeichnet, dass** das Reduktionsmittel im Nachbehandlungsmittel C ausgewählt ist aus anorganischen und organischen Schwefel-haltigen Reduktionsmitteln.

4. Verpackungseinheit (Kit-of-Parts) nach einem der Ansprüche 1 bis 3, **dadurch gekennzeichnet, dass** das Reduktionsmittel im Nachbehandlungsmittel C ausgewählt ist aus Salzen der Thiomilchsäure, insbesondere Ammoniumthiolactat, und Salzen der Dithionigen Säure, insbesondere Natriumdithionit.

5. Verpackungseinheit (Kit-of-Parts) nach einem der Ansprüche 1 bis 4, **dadurch gekennzeichnet, dass** das Reduktionsmittel im Nachbehandlungsmittel C in einem Anteil von 0,1 bis 15 Gew.-%, bevorzugt 0,5 bis 12 Gew.-%, besonders bevorzugt 1,0 bis 10 Gew.-% und insbesondere 2,0 bis 8,0 Gew.-%, jeweils bezogen auf das Gesamtgewicht des anwendungsbereiten Nachbehandlungsmittels C, enthalten ist.

6. Verpackungseinheit (Kit-of-Parts) nach einem der Ansprüche 1 bis 5 **dadurch gekennzeichnet, dass** das Nachbehandlungsmittel C zusätzlich mindestens Harnstoff enthält.

7. Verpackungseinheit (Kit-of-Parts) nach einem der Ansprüche 1 bis 6, **dadurch gekennzeichnet, dass** das Nachbehandlungsmittel C einen pH-Wert von 1,5 bis 7,0, bevorzugt 2,5 bis 6,0 und besonders bevorzugt von 3,0 bis 5,5 aufweist.

8. Verfahren zur Aufhellung keratinischer Fasern, bei dem in einem ersten Schritt eine Aufhell- und/oder Blondierzubereitung auf die Fasern aufgetragen wird, für eine Einwirkzeit T1 auf den Fasern belassen wird und anschließend mit Wasser ausgewaschen wird, und in einem zweiten Schritt ein Nachbehandlungsmittel D nach einem der Ansprüche 1 oder 3 bis 7 auf die keratinischen Fasern aufgetragen wird, für eine Einwirkzeit T2 auf den Fasern belassen wird und anschließend ausgewaschen wird, **dadurch gekennzeichnet, dass**

a. die Aufhell- und/oder Blondierzubereitung unmittelbar vor der Anwendung durch Vermischen der Oxidationsmittelzubereitung A und der Blondieraktivatorzubereitung B sowie gegebenenfalls der Alkalisierungszubereitung D gemäß einem der Ansprüche 1 oder 2 hergestellt wird und
b. der Zeitraum zwischen Beendigung des ersten Verfahrensschritt und Beginn des zweiten Verfahrensschritts maximal 60 Minuten beträgt.

9. Verfahren nach Anspruch 8, **dadurch gekennzeichnet, dass** für den ersten Schritt des Verfahrens die anwendungsbereite Aufhell- und/oder Blondierzubereitung auf einer oder mehreren Folien, geeignet zur Einschlagung einer oder mehrer Bündel keratinischer Fasern, aufgebracht ist, diese Folie und/oder Folien um ein oder mehrere Bündel keratinischer Fasern geschlagen werden, für die Einwirkzeit T1 um den Bündeln belassen werden und anschließend vor dem Auswaschen der keratinischen Fasern entfernt werden.

10. Verwendung von Reduktionsmitteln in Nachbehandlungsmitteln zur Verstärkung der Aufhellleistung bei oxidativen Aufhell- und Blondierverfahren keratinischer Fasern.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008034845 A1
- DE 10152940 A1